(19) Europäisches Patentamt

European Patent Office

Office européen des brevets

(11) EP 0 625 350 B1

(12) **EUROPÄISCHE PATENTSCHRIFT**

(45) Veröffentlichungstag und Bekanntmachung des Hinweises auf die Patenterteilung:
04.02.1998 Patentblatt 1998/06

(51) Int. Cl.$^6$: **A61K 7/09**

(21) Anmeldenummer: 94104921.5

(22) Anmeldetag: 29.03.1994

(54) **Mittel und Verfahren zur dauerhaften Haarverformung**

Composition and method for hair waving

Composition et méthode pour la déformation permanente des cheveux

(84) Benannte Vertragsstaaten:
DE ES FR GB IT

(30) Priorität: 19.05.1993 DE 4316750

(43) Veröffentlichungstag der Anmeldung:
23.11.1994 Patentblatt 1994/47

(73) Patentinhaber:
Wella Aktiengesellschaft
64274 Darmstadt (DE)

(72) Erfinder:
• Maresch, Gerhard
D-64295 Darmstadt (DE)
• Braun, Hans-Jürgen, Dr.
CH-3182 Ueberstorf (CH)

(56) Entgegenhaltungen:
US-A- 2 719 814    US-A- 4 363 815

Printed by Xerox (UK) Business Services
2.15.8/3.4

## Beschreibung

Die vorliegende Erfindung betrifft ein Mittel zur dauerhaften Haarverformung, welches als keratinreduzierenden Wirkstoff 2-Hydroxy-3-mercaptopropionsäure enthält, sowie ein Verfahren zur dauerhaften Haarverformung unter Verwendung dieses Mittels.

Bekanntlich besteht die klassische Technik zur Durchführung der dauerhaften Haarverformung darin, daß in einer ersten Stufe die Disulfidbindungen des Haarkeratins mit Hilfe eines Mittels, welches einen reduzierenden Wirkstoff enthält, (Haarverformungsmittel) geöffnet werden, sodann das Haar in die gewünschte Form gebracht wird und anschließend die Disulfidbindungen unter Verwendung eines einen oxidierenden Wirkstoff enthaltenden Mittels (Fixiermittel) wiederverknüpft werden.

Als reduzierender Wirkstoff werden hierbei insbesondere Sulfite, Thioglykolsäure, Thiomilchsäure, 3-Mercaptopropionsäure, Mercaptocarbonsäureester, Cystein und dessen Derivate oder Cysteamin und dessen Derivate verwendet.

Es ist bekannt, daß Mercaptocarbonsäureester, wie zum Beispiel der Thioglykolsäureglyzerinester, bezüglich ihrer Hautverträglichkeit sowie ihres Sensibilisierungsrisikos nicht zufriedenstellend sind, während die Thioglykolsäure eine im Vergleich zum Cystein hohe Toxizität aufweist.

Aus der US-A-4 363 815 sind kosmetische und medizinische Zubereitungen wie Shampoos und Lotionen für die Behandlung der Kopfhaut gegen Hautfunktionsstörungen, wie z. B. Kopfschuppen und Psoriasis, bekannt, welche als wirksamen Bestandteil 2-Hydroxy-3-mercaptopropionsäure in einer Menge von 0,1 bis 40 Gew.% enthalten können. Der pH-Wert dieser Zubereitungen kann im Bereich von 2 bis 6 liegen.

Es bestand daher die Aufgabe, eine neue Verwendung für möglichst natürliche - das heißt im menschlichen Organismus, beispielsweise als Stoffwechselprodukt, vorkommende - keratinreduzierende Verbindungen enthaltende Mittel aufzuzeigen sowie neue Haarverformungsmittel mit diesen keratinreduzierende Verbindungen zur Verfügung zu stellen, welche eine gute Hautverträglichkeit, ein geringes Sensibilisierungsrisiko sowie eine niedrige Toxizität und eine gute biologische Abbaubarkeit aufweisen.

Überraschen wurde nunmehr gefunden, daß durch die Verwendung von 2-Hydroxy-3-mercaptopropionsäure beziehungsweise deren Salz enthaltenden Mitteln zur dauerhaften Haarverformung eine wirkungsvolle Umformung der Haare bei gleichzeitig guter physiologischer Verträglichkeit und geringem Sensibilisierungsrisiko ermöglicht wird.

Gegenstand der vorliegenden Erfindung ist daher die Verwendung eines Mittels zur dauerhaften Verformung der Haare, welches dadurch gekennzeichnet ist, daß es als keratinreduzierenden Wirkstoff 2-Hydroxy-3-mercaptopropionsäure und/oder deren Salze enthält.

Als Salze der 2-Hydroxy-3-mercaptopropionsäure können insbesondere die Alkalisalze, das Ammoniumsalz und das Monoethanolaminsalz der 2-Hydroxy-3-mercaptopropionsäure genannt werden, wobei das Ammoniumsalz und das Monoethanolaminsalz der 2-Hydroxy-3-mercaptopropionsäure besonders bevorzugt sind.

Zwar ist es möglich die 2-Hydroxy-3-mercaptopropionsäure und/oder deren Salze gemeinsam mit anderen keratinreduzierenden Wirkstoffen - wie zum Beipiel Thioglykolsäure, Thiomilchsäure, 3-Mercaptopropionsäure, Cystein oder dessen Derivaten oder Cysteamin oder dessen Derivaten - zu verwenden, jedoch ist die Verwendung der 2-Hydroxy-3-mercaptopropionsäure und/oder deren Salzen als alleinigem keratinreduzierenden Wirkstoff (das heißt ohne Zusatz anderer keratinreduzierender Wirkstoffe) besonders bevorzugt.

Die 2-Hydroxy-3-mercaptopropionsäure und/oder deren Salze werden in dem gebrauchsfertigen Mittel zur dauerhaften Haarverformung in einer Menge von 5 bis 25 Gewichtsprozent, vorzugsweise 8 bis 20 Gewichtsprozent, eingesetzt.

Das gebrauchsfertige, erfindungsgemäß verwendete Haarverformungsmittel besitzt einen pH-Wert von 6 bis 9, vorzugsweise von 6,5 bis 8,5.

Erfindungsgegenstand ist ferner ein Mittel zur dauerhaften Verformung von Haaren, dadurch gekennzeichnet, daß es einen pH-Wert von 6,5 bis 8,5 aufweist und als keratinreduzierenden Wirkstoff 2-Hydroxy-3-mercaptopropionsäure und/oder deren Salz enthält.

Das Verformungsmittel kann sowohl in Form einer wäßrigen Lösung oder einer Emulsion als auch in verdickter Form auf wäßriger Basis, insbesondere als Gel, Creme oder Paste vorliegen.

Selbstverständlich kann das Verformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäurester; ferner Trübungsmittel, wie zum Beispiel Polyethylenglykolester; oder Alkohole, wie beispielsweise Ethanol, Propanol, Isopropanol oder Glycerin; Lösungsvermittler; Stabilisatoren; Puffersubstanzen; Parfümöle; Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von 0,2 bis 30 Gewichtsprozent, während die Verdickungsmittel in einer Menge von 0,5 bis 20 Gewichtsprozent in diesem Mittel enthalten sein können.

Weiterhin können diesem erfindungsgemäß verwendeten Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 2 bis 30 Gewichtsprozent sowie zur Vermeidung einer Überkrausung der Haare Dithioverbindungen, beispielsweise Dithiodiglykolsäure, Dithiodimilchsäure oder deren Salze, zugesetzt werden.

Durch Variation des pH-Wertes kann ein Mittel zur Verfügung gestellt werden, das universell für jede Haarstruktur, gegebenenfalls unter zusätzlicher Wärmeeinwirkung, geeignet ist. Das Mittel bewirkt eine elastische, dauerhafte und gleichmäßige Umformung vom Haaransatz bis zur Haarspitze ohne allergische oder sensibilisierende Reaktionen hervorzurufen.

Die vorliegende Erfindung betrifft weiterhin ein Verfahren zur dauerhaften Verformung von Haaren, bei dem man das Haar bevor und/oder nachdem man es in die gewünschte Form bringt, mit einem Verformungsmittel behandelt, mit Wasser spült, sodann oxidativ nachbehandelt, mit Wasser spült, gegebenenfalls zur Wasserwelle legt und sodann trocknet, welches dadurch gekennzeichnet ist, daß als Verformungsmittel das vorstehend beschriebene erfindungsgemäße Mittel verwendet wird.

Bei einer bevorzugten Ausführungsform des erfindungsgemäßen Verfahrens wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm, verwendet.

Für die oxidative Nachbehandlung kann jedes beliebige, bisher für eine derartige Behandlung verwendete, Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die nachfolgenden Beispiele sollen den Gegenstand der Erfindung näher erläutern, ohne den Gegenstand auf diese Beispiele zu beschränken.

**Beispiele**

**Beispiel 1: Dauerverformungsmittel für gefärbtes Haar**

| | |
|---|---|
| 10,0 g | 2-Hydroxy-3-mercaptopropionsäure |
| 6,1 g | Ammoniak (25%-ige wäßrige Lösung) |
| 2,0 g | Ammoniumhydrogencarbonat |
| 2,0 g | Isopropanol |
| 1,0 g | Isooctylphenol, oxethyliert mit 10 Mol Ethylenoxid |
| 1,0 g | Poly(dimethyldiallylammoniumchlorid) |
| 0,3 g | Parfümöl |
| 0,1 g | Vinylpyrrolidon/Styrol-Mischpolymerisat (Antara® 430 der GAF Corp.; New York/USA) |
| 77,5 g | Wasser |
| 100,0 g | |

Der pH-Wert dieses Mittels beträgt 8,0.

Durch Farbbehandlungen vorgeschädigtes Haar wird mit einem Shampoo gewaschen, frottiert und auf Wickler mit einem Durchmesser von 8 Millimetern gewickelt. Anschließend wird das vorstehend beschriebene Haarverformungsmittel gleichmäßig auf dem gewickelten Haar verteilt. Sodann wird das Haar mit einer Plastikhaube abgedeckt und 10 Minuten lang unter einer Trockenhaube bei einer Temperatur von 40 Grad Celsius erwärmt. Anschließend wird die Abdeckung entfernt, das Haar mit Wasser gespült und mit 100 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und sodann getrocknet.

Als Ergebnis dieser Behandlung wird eine gleichmäßige, elastische und dauerhafte Verformung der Haare erhalten.

**Beispiel 2: Dauerverformungsmittel für normales Haar**

| | |
|---|---|
| 16,0 g | 2-Hydroxy-3-mercaptopropionsäure |
| 8,9 g | Ammoniak (25%-ige wäßrige Lösung) |
| 5,0 g | Ammoniumhydrogencarbonat |
| 4,0 g | Harnstoff |
| 2,4 g | Monoethanolamin |
| 1,5 g | Isooctylphenol, oxethyliert mit 10 Mol Ethylenoxid |
| 0,5 g | Poly(dimethyldiallylammoniumchlorid) |
| 0,5 g | Parfümöl |
| 0,1 g | Vinylpyrrolidon/Styrol-Mischpolymerisat (Antara® 430 der GAF Corp.; New York/USA) |
| 61,1 g | Wasser |
| 100,0 g | |

Der pH-Wert dieses Mittels beträgt 8,4.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet.

Das so behandelte Haar besitzt eine gleichmäßige und lebhafte Krause.

**Beispiel 3: Dauerverformungsmittel für normales und schwer verformbares Haar**

| | |
|---|---|
| 3,0 g | 2-Hydroxy-3-mercaptopropionsäure |
| 15,0 g | Cysteinhydrochlorid |
| 11,0 g | Ammoniak (25%-ige wäßrige Lösung) |
| 1,5 g | Isooctylphenol, oxethyliert mit 10 Mol Ethylenoxid |
| 0,5 g | Poly(dimethyldiallylammoniumchlorid) |
| 0,5 g | Parfümöl |
| 0,1 g | Vinylpyrrolidon/Styrol-Mischpolymerisat (Antara® 430 der GAF Corp.; New York/USA) |
| 68,4 g | Wasser |
| 100,0 g | |

Der pH-Wert dieses Mittels beträgt 8,7.

Das Haar wird in der in Beispiel 2 beschriebenen Weise behandelt, wobei die Einwirkungszeit des Verformungsmittels jedoch 20 Minuten beträgt.

Als Ergebnis dieser Behandlung ist eine natürlich wirkende, gleichmäßige Umformung der Haare vom Haaransatz bis zu den Haarspitzen.

**Patentansprüche**

1. Verwendung eines Mittels zur dauerhaften Verformung von Haaren, dadurch gekennzeichnet, daß es als keratinreduzierenden Wirkstoff 2-Hydroxy-3-mercaptopropionsäure und/oder deren Salz enthält.

2. Verwendung eines Mittels nach Anspruch 1 zur dauerhaften Verformung von Haaren, dadurch gekennzeichnet, daß es die 2-Hydroxy-3-mercaptopropionsäure und/oder deren Salz in einer Menge von 5 bis 25 Gew.% enthält.

3. Verwendung eines Mittels nach Anspruch 1 oder 2, dadurch gekennzeichnet, daß das Salz der 2-Hydroxy-3-mercapto-propionsäure ausgewählt ist aus dem Ammoniumsalz und dem Monoethanolaminsalz.

4. Verwendung eines Mittels nach einem der Ansprüche 1 bis 3, dadurch gekennzeichnet, daß es die 2-Hydroxy-3-mercapto-propionsäure und/oder deren Salz als alleinigen Keratinreduzierenden Wirkstoff enthält und der pH-Wert 6 bis 9 beträgt.

5. Mittel zur dauerhaften Verformung von Haaren, dadurch gekennzeichnet, daß es einen pH-wert von 6,5 bis 8,5 aufweist und als keratinreduzierenden Wirkstoff 2-Hydroxy-3-mercaptopropionsäure und/oder deren Salz enthält.

6. Mittel nach Anspruch 5, dadurch gekennzeichnet, daß das Salz der 2-Hydroxy-3-mercapto-propionsäure ausgewählt ist aus dem Ammoniumsalz und dem Monoethanolaminsalz.

7. Mittel nach Anspruch 5 oder 6, dadurch gekennzeichnet, daß es neben 2-Hydroxy-3-mercapto-propionsäure und/oder deren Salz einen zusätzlichen keratinreduzierenden Wirkstoff, ausgewählt aus Thioglykolsäure, Thiomilchsäure, 3-Mercaptopropionsäure, Cystein oder dessen Derivaten und Cysteamin oder dessen Derivaten enthält.

8. Verfahren zur dauerhaften Verformung von Haaren, bei dem man das Haar bevor und/oder nachdem man es in die gewünschte Form bringt mit einem Verformungsmittel behandelt, mit Wasser spült, sodann oxidativ nachbehandelt, mit Wasser spült, gegebenenfalls zur Wasserwelle legt und sodann trocknet, dadurch gekennzeichnet, daß man als Verformungsmittel ein Mittel nach einem der Ansprüche 1 bis 7 verwendet.

**9.** Verfahren nach Anspruch 8, dadurch gekennzeichnet, daß man das Verformungsmittel 5 bis 30 Minuten lang einwirken läßt.

**10.** Verfahren nach Anspruch 8 oder 9, dadurch gekennzeichnet, daß man das Verformungsmittel in einer Menge von 60 bis 120 Gramm anwendet.

**Claims**

**1.** Use of an agent for the permanent shaping of hair, characterised in that it contains as keratin-reducing active substance 2-hydroxy-3-mercaptopropionic acid and/or its salts.

**2.** Use of an agent for the permanent shaping of hair according to Claim 1, characterised in that it contains an amount of from 5 to 25 % by weight of 2-hydroxy-3-mercaptopropionic acid and/or its salts.

**3.** Use of an agent according to Claim 1 or 2, characterised in that the salt of 2-hydroxy-3-mercaptopropionic acid is selected from the ammonium salt and the monoethanolamine salt of 2-hydroxy-3-mercaptopropionic acid.

**4.** Use of an agent according to any one of Claim 1 to 3, characterised in that it contains the 2-hydroxy-3-mercaptopropionic acid and/or its salts as the sole keratin-reducing active substance and the pH value is from 6 to 9.

**5.** Agent for the permanent shaping of hair, characterised in that the pH value is from 6 to 8,5 and that it contains as keratin-reducing compound 2-hydroxy-3-mercaptopropionic acid and/or its salts.

**6.** Agent according to Claim 5, characterised in that the salt of 2-hydroxy-3-mercaptopropionic acid is selected from the ammonium salt or the monoethanolamine salt.

**7.** Agent according to any one of Claims 5 to 6, characterised in that besides 2-hydroxy-3-mercaptopropionic acid and/or its salts an additional keratin-reducing compound selected from thioglycolic acid, thiolactic acid, 3-mercaptopropionic acid, cysteine or its derivatives and cysteamine or its derivatives, is contained.

**8.** Process for the permanent shaping of hair, wherein, before and/or after it is brought into the desired shape, the hair is treated with a shaping agent, rinsed with water, then treated oxidatively, rinsed with water, optionally arranged in a water wave, and then dried, characterised in that the shaping agent used is an agent according to any one of Claims 1 to 7.

**9.** Process according to Claim 8, characterised in that the shaping agent is allowed to take effect for from 5 to 30 minutes.

**10.** Process according to Claim 8 or 9, characterised in that the amount of shaping agent applied is from 60 to 120 grams.

**Revendications**

**1.** Utilisation d'une composition pour la déformation permanente des cheveux, caractérisée par le fait qu'elle contient, comme agent réducteur de la kératine, de l'acide 2-hydroxy-3-mercaptopropionique et/ou son sel.

**2.** Utilisation d'une composition pour la déformation permanente des cheveux selon la revendication 1, caractérisée par le fait qu'elle contient de l'acide 2-hydroxy-3-mercaptopropionique et/ou son sel à raison de 5 à 25 % en poids.

**3.** Utilisation d'une composition selon l'une des revendications 1 ou 2, caractérisée par le fait que le sel de l'acide 2-hydroxy-3-mercaptopropionique est choisi parmi le sel d'ammonium et le sel de monoéthanolamine.

**4.** Utilisation d'une composition selon l'une des revendications 1 à 3, caractérisée par le fait qu'elle contient, comme seul agent réducteur de la kératine, l'acide 2-hydroxy-3-mercaptopropionique et/ou son sel et que son pH est compris entre 6 et 9.

**5.** Composition de déformation permanente des cheveux, caractérisee par le fait que son pH est compris entre 6,5 et 8,5 et qu'elle contient, comme agent réducteur de la kératine, de l'acide 2-hydroxy-3-mercaptopropionique et/ou son sel.

**6.** Composition selon la revendication 5, caractérisée par le fait que le sel de l'acide 2-hydroxy-3-mercaptopropionique est choisi parmi le sel d'ammonium et le sel de monoéthanolamine.

**7.** Composition selon l'une des revendications 5 ou 6, caractérisée par le fait qu'elle contient, en plus de l'acide 2-hydroxy-3-mercaptopropionique et/ou son sel, un agent additionnel réducteur de la kératine choisi parmi les acides thioglycolique, thiolactique, 3-mercaptopropionique, la cystéine ou ses dérivés et la cystéamine ou ses dérivés.

**8.** Procédé pour la déformation permanente des cheveux selon lequel on traite les cheveux, avant et/ou après les ayant mis sous la forme souhaitée, à l'aide d'une composition de déformation permanente, on les rince à l'eau, puis on les traite à nouveau par voie oxydante, on les rince à l'eau et, le cas échéant on les met en plis, puis les sèche, caractérisée par le fait que l'on utilise comme composition de déformation permanente des cheveux une composition selon l'une des revendications 1 à 7.

9. Procédé selon la revendication 8, caractérisé par le fait qu'on laisse agir la composition pour la déformation permanente des cheveux pendant 5 à 30 minutes.

10. Procédé selon l'une des revendications 8 ou 9, caractérisé par le fait que l'on utilise la composition pour la déformation permanente à raison de 60 à 120 grammes.